# EUROPEAN PATENT APPLICATION

(11) **EP 4 481 389 A1**
(43) Date of publication of application: **25.12.2024**
(21) Application number: 23305988.0
(22) Date of filing: 21.06.2023
(51) Int. Cl.: G01N 33/68, G01N 33/72, G01N 33/92

(54) **METHOD OF DIAGNOSIS OF NON-ALCOHOLIC STEATOTIC HEPATITIS**

(71) Applicant: Biopredictive, 75007 Paris (FR)
(72) Inventor: The designation of the inventor has not yet been filed
(74) Representative: Flesselles, Bruno F.G.

(57) **Abstract**

The invention relates to a new non-invasive quantitative test making it possible to detect liver Non-Alcoholic Steatotic Hepatitis (NASH) in type-2diabetes and help identify subjects at risk of NASH using non-invasive tests that are sex-specific.

## Description

The invention relates to a new non-invasive quantitative test making it possible to detect liver Non-Alcoholic Steatotic Hepatitis (NASH) in type-2diabetes and help identify subjects at risk of NASH using non-invasive tests that are sex-specific.

Fatty liver is a reversible condition wherein large vacuoles of triglyceride fat accumulate in liver cells via the process of steatosis.

Non-alcoholic fatty liver disease (NAFLD) is one of the causes of fatty liver, occurring when fat is deposited (steatosis) in the liver due to causes other than excessive alcohol use. NAFLD is the most common liver disorder in developed countries. NAFLD is a condition generally associated with factors of the metabolic syndrome.

While a liver may remain fatty without disturbing liver function, it may also progress to become non-alcoholic steatohepatitis (NASH), a state in which steatosis is combined with inflammation and fibrosis (steatohepatitis). NASH is a progressive disease: over a 10-year period, up to 20% of patients with NASH will develop cirrhosis of the liver, and 10% will suffer death related to liver disease.

Non-alcoholic steatohepatitis (NASH) is thus the most extreme form of NAFLD, and is regarded as a major cause of cirrhosis of the liver of unknown cause.

Biopsy is the usual reference for NASH assessment. In particular, it can be used to assess severity (grade) and stage (fibrosis) of disease. This enables enrolment in clinical trials, evaluation of treatment response and may point to comorbidity risk factors. Among the usual limits of liver biopsy, especially its invasiveness, sampling variability is a challenging issue as it represents a tiny fraction (1/50 000) of the whole liver. This strongly supports the use of criteria of biopsy adequacy including length and width of liver specimen. Nevertheless, given the zonation of the disease (starting in centrolobular areas), we may expect less impact of sampling compared with viral hepatitis, for instance.

Whenever imaging tools (ultrasonography, MRE), are not available or feasible (e.g. large epidemiological studies), serum biomarkers and scores are an acceptable alternative for the diagnosis of steatosis.

The European guidelines have recommended three blood tests for the diagnosis of steatosis in large studies of subjects at risk of non-alcoholic fatty liver disease (NALFLD) and NASH (EASL J Hepatol 2015). The best-validated steatosis scores are the fatty liver index, the SteatoTest (Poynard et al 2005, Comparative Hepatology 4:10) and the NAFLD liver fat score (Angulo et al, Hepatology 2007;45(4):846-854); they have all been externally validated in the general population or in grade 3 obese persons and variably predict metabolic, hepatic and cardiovascular outcomes/mortality. These scores are associated with insulin resistance and reliably predict the presence, not the severity, of steatosis (Fedchuk et al, Aliment Pharmacol Ther 2014;40:1209-1222).

Munteanu et al (Aliment Pharmacol Ther 2016; 44:877-889) valuate the diagnostic performance of FibroTest, SteatoTest and ActiTest in patients with NAFLD using the SAF score as histological reference.

The SteatoTest is calculated using an original combination of nine biochemical markers, which are easy to assess and combines alpha2-macroglobulin, haptoglobin, apolipoprotein A1, total bilirubin, Gamma-glutamyl transpeptidase (GGT), fasting glucose, triglycerides, cholesterol, and alanine transaminase (ALT), with parameters adjusted for patient's age, gender, weight and height (Body Mass Index).

The NAFLD liver fat score uses age, BMI, IGF/ Diabetes (Yes/No), platelets, AST, ALT, and albumin (NAFLD Score = -1.675 + 0.037 × Age (years) + 0.094 × BMI (kg/m²) + 1.13 × IFG/diabetes (yes = 1, no = 0) + 0.99 × AST/ALT ratio - 0.013 × Platelet (×10⁹/L) - 0.66 × Albumin (g/dL)).

WO2014049131 relates to an in vitro non-invasive method for assessing the presence and/or severity of NASH lesions in the liver of a subject, wherein said method comprises the steps of: a. measuring in a sample of said subject at least one biomarker, preferably 1, 2, 3 or 4 biomarkers, selected from at least one of: i. at least one biomarker reflecting apoptosis, and/or ii. at least one biomarker reflecting anthropometry, and/or iii. at least one biomarker reflecting metabolic activity, and/or iv. at least one biomarker reflecting liver status; and b. combining said at least one biomarker measure in a mathematical function. The formulas disclosed on page 23-24 all include BMI. The choice of specifically disclaiming use of BMI and bilirubin is not disclosed in this document.

WO2018050804 discloses new methods for assessing NAFLD and NASH in a patient, combining measurement of serum markers through mathematical functions. The functions disclosed therein all include bilirubin.

Another test is also disclosed in WO2020021058.

These tests are all been developed in patient of the general population, and can be applied to both female and male patients, the sex being taken into consideration within the formulas.

Patients with T2D are at risk of clinically significant liver fibrosis with an increased risk of cirrhosis, primary liver cancer, and one of the most frequent causes of liver transplantation. New medical treatments are being developed for the treatment or prevention of liver fibrosis in these patients. Only bariatric surgery in obese patients has permitted to reduce the progression of fibrosis, associated with improvement of T2D. The early diagnosis of fatty liver, NASH or significant liver fibrosis cannot be performed by liver biopsy according to its risk of severe complications including death as well as the high prevalence of T2D.

The above non-invasive (NITs) tests are controversial for use for patients with T2D, as the diagnostic and performances of NITS seemed lower in indirect comparisons with those observed in viral hepatitis (Poynard et al Eur J Gastroenterol Hepatol. 2020 Aug;32(8):998-1007; Bril et al, J Investig Med. 2019 Feb;67(2):303-311).

These discordances may be due to the poor methodology of the published overviews or meta-analyses, and no may be due to the absence of prospective face to face comparisons of NITs, the high number of confounders in indirect comparisons, the absence of intention-to-diagnose methodology for imaging NITs, the use of binary AUROCs which cannot take into account the spectrum bias.

Another weakness is the frequent use of the CRN scoring system for the definition of the main endpoint, which is the clinically significant fibrosis stage with the bridging of fibrosis (The CRN system describes the nonalcoholic fatty liver disease activity score (NAS), which is a composite score of steatosis, lobular inflammation, cytological ballooning, and fibrosis (disease stage)). The CRN definition has a lower granularity for the early stages of bridging in NAFLD, for which the SAF scoring system (including the semiquantitative scoring of steatosis (S), ac- tivity (A), and fibrosis (F)) is better calibrated than the CRN score. A recent scoring system EPoS, has been described, as permitting to reduce the intra an inter-variability between pathologists definition and is better calibrated for scoring the bridging and the stages of cirrhosis (Pais et al, Hepatology. 2022 Aug;76(2):456-468).

There is thus a need to improve diagnosis methods for NASH in type 2 diabetes (T2D) patients.

The inventors have developed new tests that are optimized and well adapted for these patients, and are based on the finding that the sex of the patients is important and necessitates development of a test specific for females (bearing XX chromosomes) and another test for males (bearing XY chromosomes).

These new tests thus differ from the previous NIDs tests in that they provide outputs that are at least as accurate, and that they include biological markers that are sex-specific therefore being able to take into consideration the difference between males and females, rather that providing the same weight for biological markers whatever the sex of the patient.

The quality of a test is generally determined by drawing a Receiving Operating Characteristic (ROC) curve and measuring the Area Under Receiving Operating Characteristic curve (AUROC).

The ROC curve is drawn by plotting the sensitivity versus (1-specificity), after classification of the patients, according to the result obtained for the test, for different thresholds (from 0 to 1).

It is usually acknowledged that a ROC curve, the area under which has a value superior to 0.7, is a good predictive curve. The ROC curve has to be understood as a curve allowing prediction of the quality of a test. It is best for the AUROC to be as closed as 1 as possible, this value describing a test which is 100 % specific and sensitive. Consequently, a test with a higher AUROC than another has a "better quality" than the other.

It is reminded that
(1) sensitivity is the probability that the diagnosis is positive in individuals having the phenotype sought (detection of true positives): the test is positive if the patient is having the phenotype. The sensitivity is low when the number of false negatives is high. The sensitivity is calculated by the formula SE = (number of individuals having the phenotype in whom the sign is present)/(number of individuals having the phenotype in whom the sign is present + number of individuals having the phenotype in whom the sign is absent).
(2) specificity is the probability that the diagnosis is negative in the individuals not having the phenotype sought (non-detection of true negatives): the test is negative if the patient is not suffering from the disease. The specificity is low when the number of false positives is high. The specificity is calculated by the formula SP = (number of individuals not having the phenotype in whom the sign is absent)/(number of individuals not having the phenotype in whom the sign is absent + number of individuals not having the phenotype in whom the sign is present).
(3) Positive predictive value (PPV): is the probability of having the disease if the diagnostic test is positive (i.e. that the patient is not a false positive): the patient is having the phenotype if the test is positive. The positive predictive value is calculated by the formula PPV = (number of individuals having the phenotype in whom the sign is present)/(number of individuals having the phenotype in whom the sign is present + number of individuals not having the phenotype in whom the sign is present).
(4) Negative predictive value (NPV): is the probability of not having the disease if the diagnostic test is negative (that the patient is not a false negative): the patient is not having the phenotype if the test is negative. The negative predictive value is calculated by the formula NPV = (number of individuals not having the phenotype in whom the sign is absent)/(number of individuals not having the phenotype in whom the sign is absent + number of individuals having the phenotype in whom the sign is absent)

In order to obtain a good diagnostic test, it is important to both increase specificity and sensitivity.

Generally, a diagnosis method comprises
i. a step of gathering information from the patient
ii. a step of comparing said information with regards to thresholds
iii. a step of deducing, from the difference between the patient's information and the threshold, whether the patient has a specific disease, the stage of the patient's disease, or whether the patient's state will evolve to a given state.

As a matter of illustration
i. the information that can be gathered from the patient can be gathered directly from the patient (such as images from NMR, scanner, radiography, contrast-enhanced computed tomography), or indirectly from the patient, such as from a biological sample that has been obtained from a patient (such as urine, blood sample...). The information can be presence (or absence) and/or level of specific biological markers, whether specific from the pathogenic determinant (bacterial or viral DNA/RNA), or elevated levels of patient's markers
ii. once the information is obtained, it is compared to different values / standards and the deviation with regards to these standards is assessed. As a matter of illustration, the level of some biomarkers shall be compared to the level usually observed in healthy patients and to the levels usually observed in patients with the disease (or for patients who have been known to later evolve to a specific disease stage, for prognosis methods). Thresholds may exist, where 95 % of patients having passed the threshold have the disease and 95 % of the patients not having passed the threshold do not have the disease. For diseases where multiple clinical stages can be determined, such thresholds can discriminate the different stages. In this step ii, one may compare various types of information to their respective standards, in order to be able to reach a diagnostic in step iii (as a matter of illustration, one can use the values and information obtained from measurement of various blood or plasma markers, images from scanner and of Body Mass Index).
iii. the last step is actually making the diagnosis (resp. determining a prognosis) *i.e.* deciding whether or not the patient has the condition sought resp. whether or not the patient will evolve to a given clinical state), taking, in particular, into account the information gathered from the patient, the thresholds as described above. The physician may also take into account other elements (such as the consistency of the information gathered or the like) to make the diagnostic.

Some methods, such as the ones disclosed in the present application, shall also include a step i.a), which comprise the steps of modifying the information obtained from the patient in order to obtain a new type of information, which is the one that is then compared to the standards in step ii. Such modification is the combination of the values of variables in a function, and obtaining an end value.

It is further to be noted that the mere measurement of the values of levels of markers in the plasma or serum of a patient and the combination thereof in an algorithm or function as herein disclosed is part of a method but only provides an intermediate result (an end value or index) that would then to be compared to a reference index (threshold), in order to be able to pose the diagnostic.

It is also to be noted that the tests herein disclosed are not "gold-standard" tests, in the sense that the output (end value or index calculated by the formulas herein disclosed) is not a definitive answer as to the state of the patient. Indeed, these tests are based on statistics and there may thus be false-positive or false-negative results, which is the reason why the specific experience of the physician in interpreting the index is of importance for making the prognosis and deciding which kind of follow up is to be made to ne made for each patient.

However, due to the specificity, sensitivity, positive predictive value and negative predictive value of the tests, herein provided for various thresholds of the index, these tests are of great interest in provided a help to the physician when investigating a clinical case. Consequently, step iii as disclosed above is not direct and immediate from step ii, as the physician must interpret the result from the clinical and general context to be able to reach a conclusion.

The invention relates to an *in vitro* method for diagnosing the presence of Non-Alcoholic Steatotic Hepatitis (NASH) in a subject, wherein the subject has Type-2 Diabetes (T2D) comprising:
(a) Determining the genetic sex of the subject
(b) Obtaining the age in years of the subject
(c) Obtaining the values of biomarkers measured in the blood, plasma or serum of the subject, wherein the biomarkers are aspartate transaminases (AST), bilirubin (BILI), alpha2-macroglobulin (A2M), Glycated hemoglobin (HbAc1),
(d) Additionally obtaining the values
   ∘ of biomarker gammaglutamyl transpeptidase (GGT) when the subject is a male
(e) optionally obtaining values of other circulating biomarkers
(f) Combining the values obtained in (b), (c) (d) and (e) in a function,
(g) obtaining an output from (f), and
(h) optionally comparing the output to at least one predetermined value, and determining presence or absence of NASH based on the end value calculated in (g) and the comparison to the predetermined value(s).

Step (h) is optional, as performing steps (a) to (g) allows obtaining an intermediate result in the diagnosis method. In step (h), it is possible to diagnose whether the subject has NASH (when a predetermined value to which the output is compared is associated with a good PPV), and/or whether the subject has not NASH ((when a predetermined value to which the output is compared is associated with a good NPV).

The combination in (f) is performed by using either directly the values of the markers as measured, or after they have been transformed (generally using the value obtained after the common logarithm function (logarithm with base 10, decimal logarithm) has been applied to these values; in some embodiments, the common logarithm is applied to the sum of 1 and of the measured value (to avoid negative values)).

The function f is generally the algebraic sum of the values (whether direct or transformed as indicated above), pondered with appropriate coefficients. The coefficients are preferably the ones obtained after performance of a logistic regression as described below.

The output of (g) may be either the actual result *r* of the function combining the values as indicated above, or may be obtained after such result *r* has been normalized to be comprised between 0 and 1. Such output *o* may be *o*=1/(1+exp(*r*)). Applying such transformation to the result of the function guarantees an output between 0 and 1. It is thus easy to choose the predetermined values between 0 and 1 (for instance 0.25, 0.5 and/or 0.75).

It is to be noted that the function that is used in (f) is not the same when the subject is a male or when the subject is a female. Indeed, the inventors have shown that the relative weight of the markers (age, biological markers) is different between the sex of the subject and that is to be reflected by the use of a given function for males and another function for females, where the variability of the markers weight is reflected in the coefficients of the function.

Presence of NASH reflects activity in the liver and potentially a start of fibrosis. It is reminded that patients with NAFLD do not have liver activity or fibrosis, and that activity level generally decrease when patients enter the fibrosis status.

This method is performed *in vitro,* or *ex vivo.* It can be computer implemented, in particular when the genetic sex, the age and the values of measured biomarkers are provided to processor within a network, where the processor performs the calculation of the end value.

The values of the biomarkers are expressed in the units generally used:
(1) Age (years)
(2) alanine aminotransferase (ALT) (IU / l)
(3) apolipoprotein A-1 (ApoA1) (g / l)
(4) Aspartate transaminase (also named aspartate aminotransferase, AST) (IU / l)
(5) total bilirubin (BILI) (µmol/l)
(6) cholesterol (CT) (g/l)
(7) gamma-glutamyl transpeptidase (GGT) (IU / l)
(8) Haptoglobin (Hapto) (g / l)
(9) Triglycerides (TG or TGL) (g/l)
(10) glycosylated hemoglobin (A1c or HbA1c) (percentage)
(11) alpha2-macroglobulin (A2M) (g / l)
(12) albumin (g / l)

The method is thus specifically optimized and adapted for patients with type-2 diabetes, which is a form of diabetes mellitus characterized by high blood sugar, insulin resistance, and relative lack of insulin.

As indicated, the function in f combined various values as described in (b) to (e), and takes into account the sex of the subject obtained in (a).

Using at least the markers recited in (b) to (d), one can, in (e), use values of at least one of a marker from haptoglobin (Hapto), apolipoprotein A-1 (apoA1), alanine transaminases (ALT), triglycerides, total cholesterol, fasting glucose, γ-globulin, albumin, α1-globulin, α2-globulin, β-globulin, IL10, TGF-β1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructose-bisphosphate aldolase B, fumarylacetoacetase, transthyretin, PR02275, C-reactive protein (isoform 1), leucine-rich alpha-2-glycoprotein, serpin A11, DNA-directed RNA polymerase I subunit RPA1, obscurin (isoform 1), alpha-skeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1, and carbohydrate deficient transferrin.

Among these markers, alanine transaminases (ALT), aptoglobin (Hapto), apolipoprotein A-1 (apoA1), alanine transaminases (ALT), triglycerides, total cholesterol, fasting glucose or albumin are of particular interest.

In a particular embodiment, it is preferred when the values that are combined in the function of (f) are only values for aspartate transaminases (AST), bilirubin (BILI), alpha2-macroglobulin (A2M), Glycated hemoglobin (HbAc1) and age when the subject is a female and aspartate transaminases (AST), bilirubin (BILI), alpha2-macroglobulin (A2M), Glycated hemoglobin (HbAc1), gammaglutamyl transpeptidase (GGT) and age when the subject is a male.

As indicated above, the function sums up the various values (potentially transformed through the use of the decimal logarithm, and don not use the same coefficients or markers for male or female subjects.

The function is thus preferably in the for of
a1 + a2*Age (years) + a3*Log(AST, IU/L) + a4*Log(1 + A2m, g/L) + a5*(Hb1Ac, %) + a6*Log(Bili, µmol/L) if the subject is a female, or
b1 + b2* Age (years) + b3* Log(AST, IU/L) + b4*Log(GGT, IU/L) + b5*Log(1 + A2m, g/L) + b6*Log(Bili, µmol/L) + b7*(Hb1Ac, %) if the subject is a male.

In some embodiments, other markers are also combined to the functions.

When the function only contains the markers as indicated above, it is preferred when
▪ -12.0≤ a1 ≤-11.0, preferably -11.7≤ a1 ≤-11.3
▪ 0.015≤ a2 ≤0.025, preferably 0.018≤ a2 ≤0.024
▪ 4.30≤ a3 ≤4.60, preferably 4.40≤ a3 ≤4.50
▪ 4.30≤ a4 ≤4.50, preferably 4.30≤ a4 ≤4.45
▪ 0.17≤ a5 ≤0.32, preferably 0.20≤ a5 ≤0.30
▪ -0.90≤ a6 ≤-0.70, preferably -0.82≤ a6 ≤-0.72
▪ -12.0≤ b1 ≤-11.0, preferably -11.8≤ b1 ≤-11.4
▪ 0.0015≤ b2 ≤0.0035, preferably 0.002≤ b2 ≤0.003
▪ 2.90≤ b3 ≤3.20, preferably 3.0≤ b3 ≤3.1
▪ 1.35≤ b4 ≤1.55, preferably 1.4≤ b4 ≤1.5
▪ 2.0≤ b5 ≤2.3, preferably 2.1≤ b5 ≤2.2
▪ 0.60≤ b6 ≤0.75, preferably 0.62≤ b6 ≤0.70
▪ 0.22≤ b7 ≤0.34, preferably 0.25≤ b7 ≤0.30

In a preferred embodiment, the function is
- 11.5677 + 0.0215*Age (years) + 4.4354*Log(AST, IU/L) + 4.3850*Log(1 + A2m, g/L) + 0.2583*(Hb1Ac, %) - 0.7781*Log(Bili, µmol/L), when the subject is a female and
- 11.6213 + 0.0024* Age (years) + 3.0409* Log(AST, IU/L) + 1.4619*Log(GGT, IU/L) + 2.1631*Log(1 + A2m, g/L) + 0.6593*Log(Bili, µmol/L) + 0.2872*(Hb1Ac, %) when the subject is a male.

The coefficients of the formulas as provided above have been rounded to the 4^{th} decimal, and any function using coefficients rounded to a further or lower decimal can also be used as herein disclosed and would thus be equivalent.

In a particular embodiment, the function has been obtained by:
a) evaluating the presence of NASH in a cohort of patients with type-2 diabetes, wherein the values of circulating biochemical markers are known for the patients, and wherein the age and sex are also known
b) identifying by unidimensional analysis, the circulating biochemical markers for which the values differ significantly
   i. between the groups of male patients with NASH and male patients without NASH and/or
   ii. between the groups of female patients with NASH and female patients without NASH
c) performing a regression analysis (generally a logistic regression) for each group (male and female patients) to assess and ponder the independent discriminative value of the markers identified in step b) for the occurrence of NASH in each (male or female) studied group
d) thereby obtaining a function for male patients and/or a function for female patients, by combination of these identified independent factors.

This method made it possible to identify, for each group (male or female T2D subjects), markers that are of interest, and their relative weight, and thus to obtain a function that is applicable to T2D female subjects and another function that is applicable to T2D male subjects. The function described above have been obtained using, in particular, aspartate transaminases (AST), bilirubin (BILI), alpha2-macroglobulin (A2M), Glycated hemoglobin (HbAc1), GGT and Age as variables (for instance, GGT was not found to have a real discriminative value in the female group in (c), and the influence of BILI is difference between the groups). Use of a large number of markers in (a) would likely increase the number of markers that are eventually combined in (c), and potentially improve the quality (AUROC) of the function. It may improve the sensitivity, specificity, NPV and/or PPV. A further explanation of this process used to obtain the diagnosis function is also developed below.

As indicated, it is possible to normalize the result of the function obtained in (f) to obtain a final value comprised between 0 and 1, as this would make it easier to define predetermined values (thresholds or cutoffs) to determine whether a subject has or has not NASH.

As indicated above, the predetermined values are calculated and determined by the person skilled in the art according to the Positive Predictive Value and/or Negative Predictive Value that is desired. It is to be noted that the functions are semi-quantitative, and that the higher the result, the more NASH (activity) in the liver of the patient (the higher the risk of the patient to have NASH).

One can thus choose a first predetermined value under which the Negative Predictive Value is higher than 90%, preferably higher than 95 %, more preferably higher than 98 %. Consequently, any end value lower than this first predetermined value will essentially mean that the patient has no NASH. In some embodiments, a first predetermined value is 0.25 (when the result of the function is normalized).

It is also possible to define a second predetermined value above which the Positive Predictive Value is higher than 75 %, preferably higher than 85 %, more preferably higher than 90 %. Consequently, any end value higher than this second predetermined value will mean that the patient is very likely to have NASH and that further research could be envisaged to determine whether fibrosis, a severe complications of NASH, is present. One can choose this second predetermined value at 0.50, or higher.

The method shall be used by the physician as follows:

When the test is positive (i.e. when the end value is higher than a predetermined threshold, such as 0.5 as indicated above), the physician will first search for any other causes of liver disease that could induce steatosis and that can be easily detected, namely whether the patient has alcoholic steatosis the most frequent, and viral hepatitis with specific marker (hepatitis B,C), drug induced hepatitis or genetic steatosis or infectious steatosis. If this potential cause of steatosis is discarded, the physician may also look at metabolic causes other than type-2 diabetes (such as overweight, dyslipidemia, high blood pressure, hyperuricemia...). The existence of such other cause makes it possible to finalize the diagnosis, as these causes are associated with increased fat in the liver.

It is also to be noted that the patient will generally consult the hepatologist after some abnormalities have been detected (such as abnormalities in the common liver markers, or detection of fat in the liver by echography), so that the diagnosis will be easier to be made.

The method herein disclosed can thus generally be performed with other existing tests (the Fibrotest, disclosed in WO0216949, or the NASH test disclosed in WO2018050804. Having the results of all these tests will allow the physician to make an accurate diagnosis about the liver pathology of the patient.

In summary, the end value allows to determine presence of NASH, also taking into account the general condition and clinical assessment of the patient, which is always to take into account for diagnosis methods. The method thus makes it possible to conclude about the presence or absence of NASH in the patient, be useful to conduct other examinations to diagnose the presence of associated fibrosis.

This method is particularly interesting to detect absence of NASH (when the end value is below the first predetermined value) as this allows to avoid any unnecessary exams for the patient and will guide the physician to investigate other causes if needed.

The method herein disclosed thus makes use of an *ex vivo* combination of value of different variables, in order to obtain an end-result that is indicative of the presence of NASH in a patient with type-2 diabetes. Such combination takes also into account the sex (XX for female or XY for male) of the patient, as the functions differ depending of the biological sex.

It would thus exclude any step applied on the human body of the patient. In the method herein disclosed, it is understood that the markers, the value of which is measured and used as variable in the function, are circulating proteins or natural elements that are present in the blood, plasma or serum of a patient. The values of the chosen markers shall be measured on a sample of blood, plasma or serum previously harvested, according to methods known in the art. The values are expressed in the units according to the art. However, should other units be chosen by the person skilled in the art to expressed the measured values, this would only change the coefficients within the function (in particular the logistic regression). The method would thus still be applicable.

However, in another embodiment, the invention relates to a method for prognosis of the presence of NASH in a patient, comprising the step of harvesting blood, plasma or serum from a patient, measuring the value of markers present in the harvested sample (this would amount to measure an unusual combination of specific markers to obtain the information with regards to the liver NASH status) and combining the values as measured through a function as disclosed above. The markers are as disclosed above. Thus, said method may also include the steps of obtaining (measuring) the different values used in the function (preferably the logistic regression), such as the steps of measuring, in a blood or a plasma sample that have previously been harvested from the patient, the concentration / values / quantities of the various biological markers as mentioned above.

As disclosed, said method may also include the steps of comparing the first index (or end result obtained after combination in the function and potential normalization) to one or more predetermined threshold, as indicated above, in particular to determine whether it is higher or lower than said threshold.

Said method may also include the step of deducing the presence of NASH in said patient if the first index is above an adequate threshold, or deducing absence of liver NASH if the first index is above below an adequate threshold.

The test as designed here is a quantitative test, and preferably with the end-result value normalized and ranging from 0 to 1. It is considered that a patient is likely to present NASH (as explained above) and would thus need a treatment or follow-up when the end-result is equal or above 0.25, or to 0.50.

In the above-specified methods, the first predetermined value has been designed to be 0.25 (or 0.50), and the patient has NASH if the end-result is higher or equal to 0.25 (or 0.50).

In one embodiment, for an end value under 0.25, the patient has no NASH. For an end value between 0.25 and 0.50, there needs to be a follow-up for the patient with new evaluations on a regular basis. For an end value between 0.50 and 0.75, there is clinical significance (presence of NASH) and a treatment should be made (starting with diet and exercise). Above 0.75, the NASH is quite developed and strong treatment should be started, the patient having likely other hepatic problems.

The invention also includes a device for diagnosis of NASH in a patient, comprising:
a) a first means, wherein the first means provides a first index by combining the values as measured from markers present in the serum or plasma of a T2D patient through a function, wherein said first function is
   a1 + a2*Age (years) + a3*Log(AST, IU/L) + a4*Log(1 + A2m, g/L) + a5*(Hb1Ac, %) + a6*Log(Bili, µmol/L) if the subject is a female, or
   b1 + b2* Age (years) + b3* Log(AST, IU/L) + b4*Log(GGT, IU/L) + b5*Log(1 + A2m, g/L) + b6*Log(Bili, µmol/L) + b7*(Hb1Ac, %) if the subject is a male.

Preferably,
▪ -12.0≤ a1 ≤-11.0, preferably -11.7≤ a1 ≤-11.3
▪ 0.015≤ a2 ≤0.025, preferably 0.018≤ a2 ≤0.024
▪ 4.30≤ a3 ≤4.60, preferably 4.40≤ a3 ≤4.50
▪ 4.30≤ a4 ≤4.50, preferably 4.30≤ a4 ≤4.45
▪ 0.17≤ a5 ≤0.32, preferably 0.20≤ a5 ≤0.30
▪ -0.90≤ a6 ≤-0.70, preferably -0.82≤ a6 ≤-0.72
▪ -12.0≤ b1 ≤-11.0, preferably -11.8≤ b1 ≤-11.4
▪ 0.0015≤ b2 ≤0.0035, preferably 0.002≤ b2 ≤0.003
▪ 2.90≤ b3 ≤3.20, preferably 3.0≤ b3 ≤3.1
▪ 1.35≤ b4 ≤1.55, preferably 1.4≤ b4 ≤1.5
▪ 2.0≤ b5 ≤2.3, preferably 2.1≤ b5 ≤2.2
▪ 0.60≤ b6 ≤0.75, preferably 0.62≤ b6 ≤0.70
▪ 0.22≤ b7 ≤0.34, preferably 0.25≤ b7 ≤0.30

In a preferred embodiment, the function is
- 11.5677 + 0.0215*Age (years) + 4.4354*Log(AST, IU/L) + 4.3850*Log(1 + A2m, g/L) + 0.2583*(Hb1Ac, %) - 0.7781*Log(Bili, µmol/L), when the subject is a female and
- 11.6213 + 0.0024* Age (years) + 3.0409* Log(AST, IU/L) + 1.4619*Log(GGT, IU/L) + 2.1631*Log(1 + A2m, g/L) + 0.6593*Log(Bili, µmol/L) + 0.2872*(Hb1Ac, %) when the subject is a male.

Said first index can be later used to determine the presence of NASH in said patient and whether it is necessary to initiate treatment or follow-up, in particular using the information provided above.

In a specific embodiment, the first mean is computerized. It may be an electronic spreadsheet with the formula recorded within, that provides the first index as an output when entering the various elements mentioned above. It can also be a computer program that provides the first index as an output after receipt of the various elements mentioned above.

The first means can present one or more of the following, in either combination:
- Operate within a private or public network
- Receive the inputs (values of the various elements mentioned above) from a sender that is in a remote place (i.e. they are sent to the first means from a different location that where the first means is located)
- Require the sender to identify himself before sending the inputs
- Receive the inputs (values of the various elements mentioned above) from a secure manner
- Send the output (first index) to the sender of the inputs
- Store the output in a database (possibly with a unique identifier, making it possible to assign inputs, outputs to this identifier)
- Provides the first index with further information (such as sensitivity and/or specificity and/or positive predictive value and/or negative predictive value linked to the prevalence of the condition in the population to which belongs the patient)

Is also foreseen a non-transitory computer readable storage medium, having stored thereon a computer program comprising program instructions, the computer program being loadable into a data-processing unit and adapted to cause the data-processing unit to carry out a method for calculating a first index by combining the values as measured from markers present in the serum or plasma of a patient through a function as disclosed above, when the computer program is run by the data-processing device.

In another embodiment, the invention relates to a microprocessor comprising a computer algorithm to perform a method of determining the presence of NASH in a patient: providing the values of blood markers of a patient and optionally the age and a value allocated to the sex of the patient; and combining the values through a function (as disclosed above, or obtained as disclosed above) to obtain a score (the end-result or first index) useful for presence of NASH in the patient.

The invention also relates to the use of the functions, devices and/or microprocessors in order to make a diagnosis of presence of NASH in a patient by use of the functions and comparing the result to a predetermined threshold to determine the presence of NASH in the patient.

The invention also relates to an *ex vivo* method for diagnosis a liver disease in a patient, comprising the steps of
- Performing the method herein disclosed to determine presence of NASH in a patient
- Determining the presence of liver fibrosis in the patient, and/or
- further determining the presence of liver inflammation in the patient, and
   diagnosing a liver disease (NAFLD, NASH or severe fibrosis/cirrhosis) in the patient depending on the results of the above determinations.

It is preferred when liver fibrosis is determined by use of the Fibrotest^{®}. In other embodiments, liver fibrosis is determined by *ex vivo* measurement of liver stiffness by Vibration Controlled Transient Elastography.

In some embodiments, presence of liver inflammation in the patient is determined or confirmed by the combination of the values of biological markers in a function, in particular using the NASH test disclosed in WO2018050804 or in WO2020021058.

Devices for performing the method are also subject of the invention.

In particular, the invention also relates to methods and device to detect a liver disease in a patient, comprising the steps of
- performing the method for detecting NASH herein disclose,
- performing the method for determining liver fibrosis (Fibrotest^{®}) disclosed in WO0216949,
- performing the method for determining inflammation (NASH test disclosed in WO2018050804 or in WO2020021058),
and concluding about the liver disease depending on the results obtained for each method.

The Fibrotest^{®} combines five markers: alpha2-macroglobulin, haptoglobin, apolipoprotein A1, total bilirubin and gamma-glutamyl transpeptidase (GGT), and adjusts with sex and age. The algorithm for Fibrotest^{®} reads as follows: 4.467 x Log(Alpha2Macroglobulin (g/l)) - 1.357 x Log(Haptoglobin (g/l)) + 1.017 x Log(GGT (IU/l)) + 0.0281 x Age (in years) + 1.737 x Log(Bilirubin (µmol/l)) - 1.184 x ApoA1 (g/l) + 0.301 x Sex (female=0, male=1) -5.540. It allows to determine the state of fibrosis (F2 or higher according to the METAVIR classification).

The NASH test is in the form of a1 + a2 x Log (A2M, g/l) + a3 x Age (years) + a4 x Log (ALT, IU / l) + a5 x (Apoa1, g/l) + a6 x Log (AST, IU/l) + a7 x Log (BILI, µmol/l) + a8 x Log (CT, mmol/l) + a9 x Gender (0 for women, 1 for men) + a10 x Log(GGT, IU / l) + a11 x Log (Hapto, g/l) + a12 x Log (TG, mmol/l)
with
- -8 ≤ a1 ≤ -7
- 0.1 ≤ a2 ≤ 0.6 preferably 0.15 ≤ a2 ≤ 0.55
- 0.02 ≤ a3 ≤ 0.05 preferably 0.03 ≤ a3 ≤ 0.04
- 1.1 ≤ a4 ≤ 1.5 preferably 1.2 ≤ a4 ≤ 1.4
- -0.2 ≤ a5 ≤ 1.0
- 1.8 ≤ a6 ≤ 2.3 preferably 1.95 ≤ a6 ≤ 2.2
- 0.8 ≤ a7 ≤ 1.6 preferably 0.9 ≤ a7 ≤ 1.5
- -1.7 ≤ a8 ≤ -1.3 preferably -1.6 ≤ a8 ≤ -1.4
- 0.015 ≤ a9 ≤ 0.20
- 0.15 ≤ a10 ≤ 0.25 preferably 0.20≤ a10 ≤ 0.22
- -0.3 ≤ a11 ≤ 0.1
- 0.9 ≤ a12 ≤ 1.2 preferably 1.0 ≤ a12 ≤ 1.1

This NASH test allows to detect inflammation when the end-result (normalized) is higher than 0.25.

WO2020/021058 discloses a method for diagnosis steatosis, using a function that is 5.17 - 0.022 x Age (years) + 1.02 x ApoA1 (g/l) + 1.99 x Log (A2M, g/l) - 1.16 x Log(GGT, IU / l) - 1.33 x Log(ALT, IU / l) + 0.6 x Log(AST, IU / l) - 0.29 x Log (Hapto, g/l) - 1.35 x Log (Triglycerides TG, mmol/l) - 0.68 x Log (Total Cholesterol CT, mmol/l) - 3.46 x Log (Fasting glucose, g/l) + 0.46 x Gender (0 for women, 1 for men).

WO2018050804 discloses a function to measure severity of NAFLD, that is - 25.98652 + 16.00374 x Log(Alpha2Macroglobulin (g/l)) + 0.10067 x Age (in years) + 1.12881 x Log (ALT, IU / l) - 4.24187 x ApoA1 (g/l) + 2.55422 x Log (AST, IU / l) + 6.22308 x Log(Bilirubin (µmol/l)) + 0.59340 x Log (CT, mmol/l) + 1.07838 x Sex (female=0, male=1) + 3.64357 x Log(GGT (IU/I)) - 4.86167 x Log(Haptoglobin (g/l)) + 1.11641 Log (TG, mmol/l).

These tests are known in the art and their use is not further developed herein.

It is also possible to use function to detect steatosis (NAFLD) or fibrosis, and that are adapted and optimized to T2D patients, and that are sex-specific (different depending on the sex/gender of the patient).

As indicated above, the function used in the method herein disclosed is a function obtained by logistic regression (which can be called logistic function).

Methods to perform logistic regressions are known in the art. In summary, they consist in evaluating the individual variations of markers between populations of subjects (one with the output such as NASH and the other where the output is absent). The markers which are the most variable can be chosen and logistic regression analysis is made to ponder the independent discriminative value of the selected markers. If some markers do not vary between groups, the coefficients of these markers in the function will be low (thereby indicating that the weight of these markers is of no real influence for the presence of the phenotype). In the context herein, regression is performed on T2D patients, and one is performed on the subgroup of female patients, and another one is performed on the subgroup of male patients.

The invention thus also pertains to a method for obtaining a function for identifying the presence (or absence) of NASH (or diagnosing NASH) in a patient wherein said function combines the values of the concentration of biochemical markers in the blood / serum or plasma of said patient and the age, comprising:
a) evaluating the presence of NASH in a cohort of patients with type-2 diabetes, wherein the values of circulating biochemical markers are known for the patients, wherein the cohort of patients contains only male patients or only female patients (all patients have the same gender)
b) identifying by unidimensional analysis, the circulating biochemical markers for which the values differ significantly between the groups of
   i. patients with NASH and
   ii. patients without NASH
c) performing a logistic regression analysis to assess and ponder the independent discriminative value of the markers identified in step b) and of the age of the patients for the occurrence of NASH,
d) thereby obtaining the function, by combination of these identified independent factors, wherein the function allows for diagnosing the presence of NASH in a subject.

This function will thus be specific for the patients of the same sex than the one of the group on which the regression has been performed, and is particularly adapted for T2D patients.

It is possible to perform the above method twice, one time with a group of male T2D patients and another time with a group of female T2D patients, thus obtaining two functions.

In the method for developing such diagnostic test, one would prefer one or more of the following, in either combination:
(a) in step a), to use a test cohort comprising at least 100 patients.
(b) In the cohort of a), that at least 10% of the patients are patients who have no NASH and no activity (negative control)
(c) The biochemical markers of step (a) are selected from the group consisting of α2-macroglobulin (A2M), GGT (gammaglutamyl transpeptidase), haptoglobin, apolipoprotein A-I (apoA1), alanine transaminases (ALT), aspartate transaminases (AST), triglycerides, total cholesterol, fasting glucose, γ-globulin, albumin, α1-globulin, α2-globulin, β-globulin, IL10, TGF-β1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructose-bisphosphate aldolase B, fumarylacetoacetase, transthyretin, PR02275, C-reactive protein (isoform 1), leucine-rich alpha-2-glycoprotein, serpin A11, DNA-directed RNA polymerase I subunit RPA1, obscurin (isoform 1), alpha-skeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1, glycosylated hemoglobin (Hb1Ac) and carbohydrate deficient transferrin
(d) AST, A2m, Hb1Ac, Bilirubin, and optionally GGT are used in the obtained function
(e) at least 3, more preferably at least 4 are used in the mathematical function
(f) at most 6, more preferably at most 5 biochemical markers are used in the mathematical function
(g) The method further comprises a step of validating the logistic function on a validation cohort comprising at least 100 patients.

In order to obtain a function that is as accurate as possible, the number of patients in the cohort should be as large as possible, indicating that it preferably comprises more than 50 patients, preferably more than 100 patients, preferably more than 200 patients, more preferably more than 500 patients, or even more than 1000 patients. As indicated, there is no upper limit for the number of patients, and the larger, the better.

Once NASH has been diagnosed in a patient, the physician can start and provide an appropriate diagnostic of associated severe disease, inflammation (NASH) or fibrosis. The invention also relates to a method for treating a patient having a NASH, for which there is a need to confirm presence of NASH, comprising the steps of performing the method of diagnosis as disclosed above and providing the patient with appropriate treatment depending on its level of NASH. In another embodiment, the invention provides a method for treating a patient, comprising the steps of providing appropriate NASH treatment to a patient depending on its level of NASH. Such treatment should in particular be performed when the end value calculated by the method as disclosed above is higher than 0.25, preferably higher than 0.50.

The primary treatment for patient with NASH will be exercise and/or a dietary treatment in order to manage obesity, high blood sugar, high blood lipids and excess iron levels if present. Alcohol should also be avoided. It is also intended to have a weight loss of the patient. Adapting the type-2 diabetes treatment of the patient is also contemplated.

As treatments or other actions that can be used in methods of treating a patient in need thereof (with NASH as determined by the methods herein disclosed), one can thus cite having the patient exercise, performing bariatric surgery, performing a biopsy for determination of NASH and/or fibrosis, performing a NIT for determination of NASH and/or fibrosis.

Medicinal drug can also be provided to the patient with NASH or fibrosis. As an illustration, one can cite administration of vitamin E (essentially for patients without cirrhosis), thiazolidinediones (TZDs, like pioglitazone), glucagon-like peptide-1 receptor agonist (GLP-1 RA, such as liraglutide, semaglutide) and sodium-glucose cotransporter-(SGLT)-2 inhibitors (such as ipragliflozin, dapagliflozin or empagliflozin).

Other drugs can also be used, such as ethyl-eicosapentanoic acid or ethylicosapentate (Clinical Trials NCT01154985), simtuzumab (GS 6624) (Clinical Trials, NCT01672866 and NCT01672879), GFT 505 (Clinical Trials NCT01694849), liraglutide (Clinical Trials NCT01237119), losartan (Clinical Trials NCT01051219), cenicriviroc (Clinical Trials NTC002217475), selonsertib and aramchol, the thyroid hormone receptor beta (THR-β) agonist resmetirom.

efruxifermin(FGF-21), pegbelfirmin (FGF-21), aldafermin (FGF-19), pegozafermin (FGF-21), BFK8588A (FGF-21), MET-409 (FXR agonist), pegozafermin, efruxifermin, VK2809 (THR-beta agonist) are also worth considering.

One can also cite selective farnesoid X receptor (FXR) agonist with anti-cholestatic and hepato-protective properties, such as obeticholic acid (OCA), which is also useful for treating fibrosis, and improves the histological features of non-alcoholic steatohepatitis..

For treating NASH and fibrosis, one can also cite lanifibranor, pirfenidone (PFD), and cenicriviroc (CVC). Lanifibranor activates each of the three PPAR isoforms. PFD is an oral antifibrotic drug efficacious and safe in patients with advanced liver disease. CVC targets macrophages in the liver by inhibiting the C-C chemokine receptors CCR2 and CCR5 that showed efficacy in treating fibrosis, and have some interest for NASH patients with F2 or F3 fibrosis.

It is also possible to consider selonsertib that has been demonstrated some activity in patients with moderate-to-severe NASH to reduce fibrosis, activity, inflammation, and progression to cirrhosis. Simtuzumab, a monoclonal antibody against lysyl oxidase-like 2 may also be considered. On can also cite the galactin-3 inhibitor GR-MD-02, elafibranor.

GLA (Gut Liver Axis) may be a target, using gut-microbiome modulators, Platelet-derived growth factor (PDGF) blockers, in particular PDGFR kinase activity blockers. As an illustration, imatinib mesylate (Gleevec^{®}) inhibits PDGFR signaling in mice. One can also consider Wnt3a, a canonical Wnt ligand which has been shown to present an ant-inflammatory role.

This method is particularly interesting in that it makes it possible to manage the follow-up of the patient, and the level of NASH in the patient, and propose further diagnosis tests (such as confirming the presence of NASH by biopsy, or of fibrosis, or perform biopsy) only to a fraction of patients that are not improving over time.

The invention also includes a device for diagnosis of NASH in a patient, comprising a first means, wherein the first means provides a first index by performing a method as described above.

Said first index can be later used to determine presence of NASH in said patient and whether it is necessary to initiate treatment or follow-up, or perform further investigations.

The above method can be computer-implemented. In this embodiment, it is possible to perform a method for determining whether a patient has NASH, comprising
a) requiring a sender to identify himself to a system within a public or private network, wherein the sender is generally in a place remote from the system
b) requiring the sender to fill in information related to the patient (such as social insurance number, sex, birth date, weight, height), and assigning a specific identifier to the patient in a database, (this last step may be omitted if the patient has already been recorded in the database, as can be determined, using the patient information);
c) receiving values of the concentration of measured biological markers, in particular (AST, A2M, Hb1Ac, BILI, GGT, Age (it may be calculated from the birth date), wherein the values are received in a secure manner, and storing the values in the database, associated with the specific identifier of the patient
d) combining the values received or calculated in c) by use of a function as disclosed herein, obtaining a result, and optionally normalizing the result
e) storing the result optionally normalized of d) in a database associated with the specific identifier and preferably the date and time of identification of the sender, thereby obtaining a database where the information related to the patient is present under the specific identifier,
f) sending the nature of the presence of NASH to the sender, wherein the patient has NASH if the result is above a predetermined value and the patient has not NASH if the result is below the or another predetermined value.

In a first step, a sender is required to identify himself to a server or system within a public or private network. It is preferred when the network is a private network, and when the connection to the server is encrypted. Identification of the sender can be performed using login and password, preferably through strong identification processes (such as one-time password, or using biometric or smart card identification).

Upon login to the server, the sender is required to fill-in information related to the patient (such as name, sex, birth date, height, weight, social security identification number, social insurance number) and a specific identifier is assigned to the patient in a database. If the database does not contain any entry relating to the patient, one entry is created. If an entry already exists (using the social security identification number allows ensuring the unicity of entry), such entry is activated.

The server shall then receive values of the concentration of biochemical markers as disclosed herein, in particular (AST, A2M, Hb1Ac, BILI, GGT, Age (it may be calculated from the birth date), and store them in a database, associated with the specific identifier of the patient, so that such data is uniquely associated to the patient. Exchanges between the sender's device and the server are very advantageously performed in a secure manner (encrypted exchanges). Age, sex and BMI (Body Mass Index) of the patient are to be sent or may be calculated using the information related to the patient. Such information is also preferably received in a secure manner, and stored in the database, associated with the specific identifier of the patient.

It is to be noted that the sender is generally in a remote place different from where the server is located. It is also envisaged that the information is recovered by the server, using information provided by the sender. For instance, the server may open a connection with the server of the laboratory having measured the biochemical markers, using identifiers provided by the sender, to upload the required information.

The next step is to combine the markers in a function as disclosed herein to obtain a result, that is optionally normalized as disclosed. The function that is used is specific according to the patient's sex.

The information pertaining to the comparison is then stored in a database associated with the identifier specific of the patient and is preferably date and time-stamped. Indeed, since the method is to be performed on a regular basis, it may be interested to keep a record of the results obtained overtime. Thereby, a database is obtained, containing identification related to the patient (patient's specific identifier), associated with the biochemical data, and comparison information.

The nature of the NASH status of the patient (presence of absence) is thus determined according to the methods herein disclosed.

Such information (nature of the risk of the patient) is then transmitted to the sender, optionally with the result of the function and with any other potential information of interest.

The invention also relates to a computer-implemented method for diagnosing the presence of NASH in a subject, wherein the subject has Type-2 Diabetes (T2D) comprising
a) Receiving inputs from a sender, wherein the inputs are the values of biomarkers measured in the blood, serum or plasma of the subject, as well as the information pertaining to the age, height, weight and sex of the subject
b) Performing the method as disclosed herein, to obtain an output (end-result) for the combination of the values received in (a), optionally normalized
c) optionally comparing the output to a reference value and determining the presence of the NASH
d) Sending the output to the sender and the presence of NASH, if determined, and optionally further information (such as sensitivity and/or specificity and/or positive predictive value and/or negative predictive value linked to the prevalence of the condition in the population to which belongs the patient)
e) Optionally storing the inputs and the output in a database (preferably with a unique identifier associated with the patient and/or with the sender, making it possible to assign inputs, outputs to this identifier)

The invention also relates to a method for obtaining the information pertaining to the presence of NASH in a subject, wherein the subject has Type-2 Diabetes (T2D) comprising
a) Sending inputs to a remote server that is in a remote place (i.e. they are sent to the first server that is in a different location than the location of the sender), preferably in a secure [encrypted] manner, wherein the inputs are the values of biomarkers measured in the blood, serum or plasma of the patient, as well as the information pertaining to the age, sex, height and weight of the patient
b) Having the remote server combine the values received in (a) in a function, according to the methods herein disclosed, and obtain an output [end result], optionally normalized
c) optionally having the remote server compare the output to a reference value and determining the presence or absence of NASH in the subject
d) Optionally having the remote server store the inputs and the output in a database (possibly with a unique identifier associated with the subject and/or with the sender, making it possible to assign inputs, outputs to this identifier)
e) receiving the output from the server and/or the information pertaining to the presence or absence of NASH (when determined) and optionally further information (such as sensitivity and/or specificity and/or positive predictive value and/or negative predictive value linked to the prevalence of the condition in the population to which belongs the subject).

In another embodiment, the invention relates to a microprocessor comprising a computer algorithm to perform a method for calculating the presence or absence of NASH from the markers present in the serum or plasma of a patient as disclosed through a method as disclosed above.

The invention also relates to the use of the functions, devices and/or microprocessors in order to assess presence or absence of NASH by use of the methods herein disclosed.

The following examples are meant to describe an aspect of invention, but shall not be limiting the invention.

### Description of the figures

Figure 1: Flow chart of participants.
Figure 2: Diagnostic performances assessed by intention-to-diagnose or standard method.

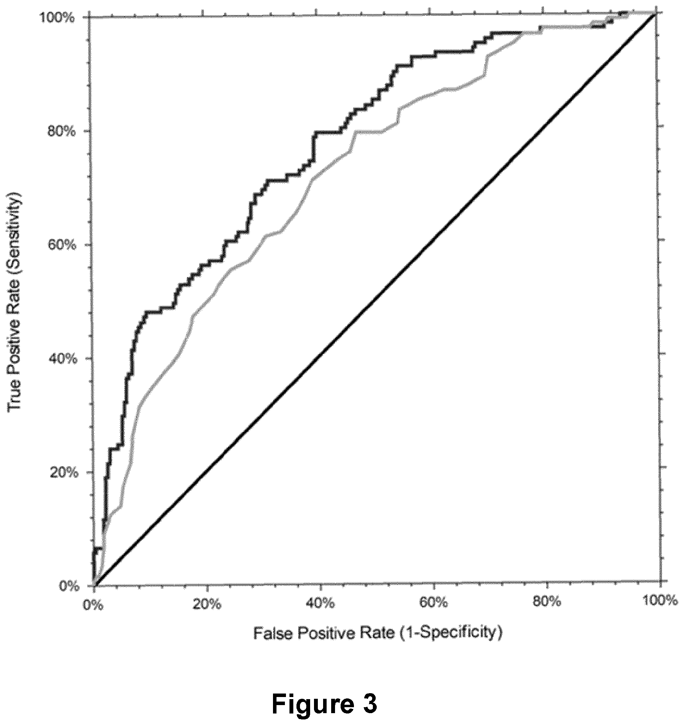

Figure 3: comparison of AUROC of the NashTest-T2D according to the invention (upper curve) and the NashTest of the prior art (bottom curve).

### Examples

### Abbreviations

AST: Aspartate transaminase or aspartate aminotransferase,
AUROC: Area Under Receiving Operating Curve
CAP: controlled attenuation parameter
EPoS staging system: a reproducible 7-tierfibrosis score for NAFLD adapted both to glass slides and digitized images
FIB4: screening test for liver fibrosis, calculated from platelet count, patient age and transaminases, easily available free of charge on the Internet.
HRG: hepato-renal gradient
NAFLD: non-alcoholic fatty liver disease
NASH: non-alcoholic steatohepatitis
NIT: noninvasive liver test
T2D: type-2 diabetes
SAF test: Steatosis Activity Fibrosis test
VCTE: vibration-controlled-transient-elastography stiffness
2dSWE: two-dimensional-share-ware-elastography stiffness

### Example 1. Summary and conclusions

Background: No prospective diagnostic studies have been published comparing directly widespread noninvasive liver tests (NITs) in patients with type-2 diabetes (T2D), and using intention-to-diagnose method, for each of the three main histological liver features, fibrosis, non-alcoholic steatohepatitis (NASH) and steatosis (NAFLD).

Aims: We compared the diagnostic performance of nine NITs using the intention-to-diagnose (n=402) and not the standard method which would rule-out not-evaluable participants (n=55).

Method: The reference used concomitant biopsy, with predetermined cutoffs scores of advanced features:
Fibrosis was considered using the EPoS fibrosis bridging for stage-F3 or more, NASH was determined using the SAF score, for grade NASH-N3 or more.

NAFLD (steatosis) was determined using the SAF score for a grade steatosis-S3 or more.

The examples below describe optimization of NITs to identify fibrosis, NAFLD, or NASH in T2D patients (respectively FibroTest-T2D, SteatoTest-T2D, NashTest-T2D).

FibroTest-T2D was compared to vibration-controlled-transient-elastography stiffness (VCTE), two-dimensional-share-ware-elastography stiffness (2dSWE), and FIB4;

The NashTest-T2D was compared to transaminase AST.

The SteatoTest-T2D was compared to controlled attenuation parameter (VCTE-CAP) and the hepato-renal gradient (2dSWE-HRG) tests.

The comparisons were made using the intention-to-diagnose, which makes it possible to take into account the fact that it may not be possible to obtain a usable result with some of the reference tests herein mentioned.

Comparison was also made to the tests disclosed in WO2018050804.

Results: Out of 402 cases, non-evaluable NITs were for VCTE 6.7%(n=27), 2dSWE-HRG 4.0%(n= 16), VCTE-CAP 3.2%(n=13), 2dSWE 1.5%(n=6), NashTestT2D 1.2%(n=5;), and all .02%(n=1) for FIB4, AST, and SteatoTestT2D.

For fibrosis staging, using intention-to-diagnose, the AUROCs between FibroTest-T2D (.77), VCTE (.77) and 2dSWE (.78) were similar (all-differences<2%;P>.50) but higher than FIB4-score (.70;all-differences>=7% P<=.03).

For NASH, NashTest-T2D had higher AUROC (.76) than AST (.71) +5%(95%CI;.3-8%; P=.04).

For steatosis the AUROCs between SteatoTest-T2D (.75), VCTE-CAP (.69) and 2dSWE-HRG (0.71) were similar (all<=6%;P>0.10). It is worth to note that standard over-estimates AUROCs of VCTE for fibrosis, by 6% (.83;95%CI.78-.87 vs .77;95%.72-.81; P<.05), as compared to intention-to-diagnose analysis.

Conclusions: In T2D outpatients, using intention-to-diagnose analysis, optimized NITs FibroTest-T2D, NashTest-T2D and SteatoTest-T2D presented a quality, as represented by the AUROC, that is at least as good as tests regularly performed, and better than blood tests described in WO2018050804 for this specific T2D population.

### Example 2. Material and Methods

### Study Design and Participants

This study was nested in the QUIDNASH study (ClinicalTrial.gov identifierNCT03634098), an investigator-initiated, prospective, cross-sectional, multicenter study aimed at identifying noninvasive biomarkers of the diagnosis and severity of NASH in patients with T2D, using liver histopathology as the gold-standard. Details of methods were already described in the previous intermediate publications, the validation of the Nash-FibroTest (a panel including FibroTest, SteatoTest and NashTest) in T2D (Poynard et al, Aliment Pharmacol Ther. 2021;54:952-966) and the assessment of NASH prevalence and advanced Fibrosis in T2D in the first 330 participants (Castera et al, Diabetes Care. 2023 Apr 12:dc222048).

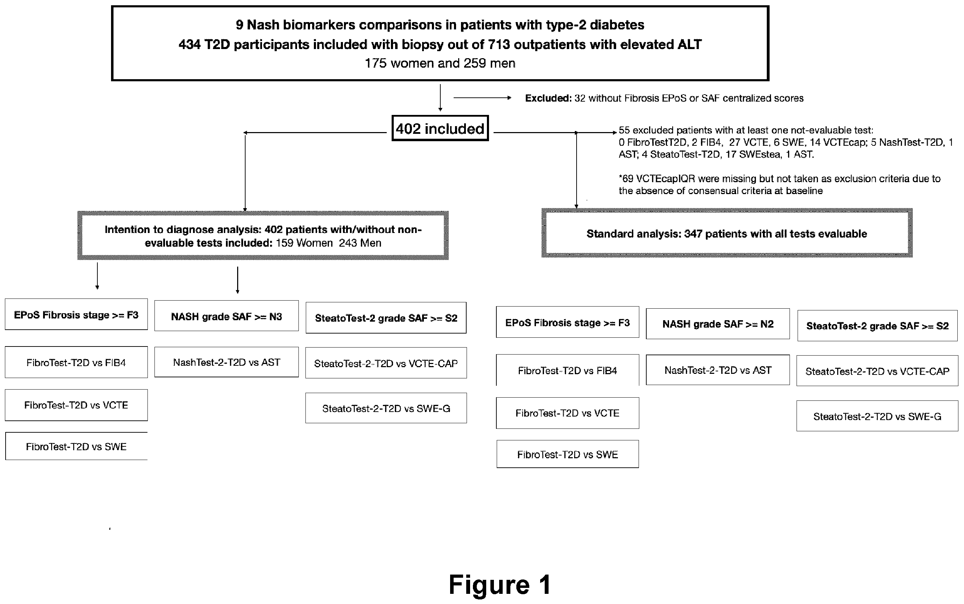

A total of 713 outpatients with T2D, systematically screened for NAFLD, were referred to hepatologists for further work-up. The present study involved 402 participants with interpretable biopsies for whom nine NITs evaluating histological features were prescribed (Figure 1).

The study was conducted in accordance with the Declaration of Helsinki and has been approved by the Research Ethics Committee (Comité de Protection Personnes Sud Méditerranée no.18.021-2018-A00311-54). Participants who had agreed to undergo a liver biopsy as part of standard of care gave written informed consent for the present study as part of the QUID-NASH study.

The project was initiated in October 2018 in four diabetes outpatient clinics across the Paris (France) area. Main inclusion criteria were as follows: 18 years of age; able to give written informed consent; and with T2D defined according to American Diabetes Association (ADA) or World Health Organization criteria, having an indication for liver biopsy which they had agreed to undergo. Exclusion criteria were as follows: pregnant women; patients without national social security coverage; patients with other causes of chronic liver disease than NASH, including excessive alcohol consumption (>20 g/day or women; >30 g/day for men); positivity for hepatitis B, hepatitis C; markers of autoimmune hepatitis, hemochromatosis; a-1 antitrypsin deficiency; Wilson disease; obstruction of liver vessels or biliary tract; patients who have undergone liver transplantation; patients with blood hemoglobin <7 g/L, or <10 g/L if they have cardiovascular or pulmonary disease; patients who refuse to undergo blood tests; patients with a possible ongoing chronic alcohol consumption as indicated by serum carbohydrate-deficient transferrin percent >2% unless explained otherwise; and patients with a confirmed diagnosis of active malignancy or other terminal diseases. Patients with suspected NAFLD were referred to a hepatologist for further workup. A liver biopsy was considered as part of standard of care based on predefined criteria: persistently elevated transaminase levels (serum ALT levels >20 IU/L in women or > 30 IU/L in men on at least two occasions) and documented absence of other causes of liver disease as detailed above.

### Aim

The aim was to compare the diagnostic performance of nine NITs.

For the fibrosis, the NashFibroTest panel was adapted (optimized) for T2D (NashFibroTest-T2D) and compared with vibration controlled transient elastography stiffness (VCTE), two-dimensional share-ware-elastography stiffness (2dSWE), and FIB4.

For the NASH: NashTest-T2D versus AST.

For the steatosis: SteatoTest-T2D versus controlled attenuation parameter (VCTE-CAP) and the hepato-renal gradient (2dSWE-HRG).

Concomitant biopsy was the reference using predetermined cut-off histological scores for advanced features: EPoS fibrosis bridging stage F3, SAF advanced grades NASH N3 and steatosis S3.

Intention-to-diagnose analysis included 402 patients, whereas standard analysis 347 patients after exclusion of 55 patients with at least one non-evaluable NITs, which include missing and non-interpretable data.

### NITs assessments

Clinical assessment, complications of diabetes, biological and NITs assessment were detailed elsewhere (Castera et al, Diabetes Care. 2023 Apr 12:dc222048). In summary, for each patient, a 12h fasting blood sample was collected and tested locally for laboratory parameters using standard formula for FIB4 (FIB-4 = Age (years) × AST (U/L) / [PLT(10⁹/L) × ALT^{1/2} (U/L)]) and manufacturers' recommendations. VCTE-CAP and LSM were performed by trained nurses or physicians and certified by the manufacturer and blinded to the patient's histological evaluation, using VCTE (FibroScan 502 Touch model; Echosens, Paris, France) equipped with both M and XL probes. The CAP and LSM results were expressed in dB/m and kPa, respectively.

### Predetermined definition of NITs effectiveness.

Effectiveness was defined as the proportion of patients with: both a non-missing result and a reliable result (Poynard et al, Med Decis Making. 1982;2:285-297). For a blood test, a missing data could be due to an impossible venous punction (missing result) or an hemolyzed serum (not reliable result), and for stiffness to less than 10 stiffness measures (missing result) or an IQR/median >=30% (not-reliable result).

For VCTE-stiffness only examinations with at least 10 valid individual measurements, as well as with interquartile range on median ratio (Stiffness IQR/median) <0.30, were deemed reliable. For VCTE-CAP, at the time of protocol construction, only examinations with at least 10 valid individual measurements were deemed reliable, without exclusion criteria due to the CAP IQR/median ratio in the absence of consensual guidelines. Recently, a study validated the reliability of CAP if IQR/median <0.30. Therefore we tested the impact of this cutoff on the CAP performance in intention-to-use in a post-hoc sensitivity analysis (Semmler et al, United European Gastroenterol J. 2020;8(3):321-331).

The 2dSWE was performed using the Aixplorer ultrasound system (Supersonic Imagine S.A.,

Aix-en-Provence, France). The medians of Qbox elasticity (stiffness) were assessed, as well as the lowest and the highest elasticity values. There is no consensus on the criteria of reliability for 2dSWE in NAFLD (Selvaraj et al, J Hepatol. 2021;75:770-785; Schuetz et al, BMJ. 2012;345:e6717). Each 2dSWE acquisition consisted of 10 sequential measurements and as previously validated in chronic liver diseases, we used a minimal value of the elasticity (0.2kPa) identified as a criterion of interpretable stiffness as well as at least six stiffness measures (Poynard etal, PLoS One. 2016;11:e0163276).

### Predetermined histological cutoffs

The predetermined cut-offs for the advanced features classes were for Fibrosis stage >=F3:
the FibroTest-T2D >=0.58, VCTE and SWE >=9.5 kPa, FIB4 >= 2.67;
for NASH grade >=N3: NashTest-T2D >= 0.50 and
for Steatosis grade >= S3: SteatoTest-T2D >=0.50.

### Histopathologic assessments

Liver biopsies were performed according to local standard procedure in the liver units of hospital. The transcapsular route, or transjugular route in case of concomitant anticoagulant treatment or morbid obesity, was used. Slides were analyzed in each center as part of standard care and then centrally reviewed by a single expert pathologist (P.B.), blinded to all patient characteristics, for the readouts. NASH was diagnosed using the Fatty Liver Inhibition of Progression (FLIP) definition (i.e., presence of steatosis, hepatocyte ballooning, and lobular inflammation with at least 1 point for each category). Biopsy specimens were categorized by the pathologists as NAFL (isolated steatosis) or NASH.

To provide more granularity for fibrosis stages, the new EPoS seven-tier classification was used as follows: F0, normal; F1: NASH-CRN stages 1a, 1b, and 1c; F2, central or portal fibrosis + lobular fibrosis or portal + central fibrosis; F3, bridging with few septa (≤2/10-mm biopsy length); F4, bridging with numerous septa (>2/10-mm biopsy length); F5, many septa with few nodules (incomplete cirrhosis with nascent regenerative nodules); and F6, annular fibrosis with complete nodulation.²⁷

### Post-hoc sensitivity analyses

The impact of a recent reliability definition of the 2dSWE-CAP reliability as an IQR/median <0.30 was assessed in intention-to-use (Semmler et al, op.cit.).

The performance of a recent test designed for "Fibrotic NASH" [FINI Index] combining Nash grade CRN>=N2 and Fibrosis stage CRN>=F2 combining including HbA1c, AST and HDL-cholesterol (Tavaglione et al, Clin Gastroenterol Hepatol. 2023;21(6):1523-1532.e1), was also assessed in a post-hoc analysis, for comparing its performance head-to-head to FibroTest-T2D and NashTest-2D.

### Statistical Methods

The analysis population was defined as all patients included in the QUID-NASH project who had an interpretable liver biopsy specimen. Qualitative data were described by absolute (n) and relative (%) frequencies. Quantitative data were described by median and interquartile range.

The optimizations of FibroTest-T2D, NashTest-T2D and SteatoTest-T2D were assessed in comparison with previous generations, named here FibroTest, NashTest, and SteatoTest, using a significant difference between AUROCs for the advanced pre-determined 0.5 cutoff given by the bootstrapping stepwise logistic for the diagnostic of each liver features as the main endoint.

The first principle was to use separate logistic regressions in women and men for more personalized algorithms.

The second was to use in the regression all the eleven components present in the previous generation of the NashFibroTest combo (as described in WO2018050804) for which the risk of false positive/negative are well known.

The third principle was to assess the possible additive discriminant value of the widely available glycated hemoglobin (HbA1c), with epidemiologic studies demonstrating robust links with complications in patients with T2D.

The following criterion were also assessed: true positive, true negative, false positive, false negative, sensitivity (Se), specificity (Sp), positive predictive value (PPV) and negative predictive value (NPV).

Then the diagnostic performances of the optimized tests were assessed both in intention-to-diagnose and in standard analyses. Standard analyses were performed after exclusion of missing or predetermined non-reliable results. Standard method performance of the diagnostic using the classical four-cell matrix: true positive, true negative, false positive, false negative. Intention to treat analysis introduces the effectiveness of a test assessed using a six-cell matrix defined as: the proportion of test ((true positives + true negatives) / (true positives + true negatives + missing + non-interpretable results)) (Poynard et al, Med Decis Making. 1982;2:285-297; Simel et al, Med Decis Making 1987;7:107-14). In intention-to-diagnose, the diagnostic performance is assessed in a "worst case scenario": non evaluable cases with a positive reference (here the presence of advanced feature) were counted towards false positives and non-evaluable cases with a negative reference (absence of advanced feature) were counted towards false negatives, and then presented in six-cell matrix accordingly.

Then, for the missing or non-interpretable NITs data, we generated an adapted AUROC using "worst case scenario" values. Indeed for calculating an AUROC, individual data are required, and by definition, missing data or not-reliable data are missing. So, missing or not-reliable values were replaced according to the worst scenario: false positive was defined as the 25^{th} centiles value of reliable values, and false negative defined as the 75^{th} centiles value of reliable values, from each sex distribution. For example, a man with a stage F3 biopsy and with less than 10 measurements of VCTE-stiffness (not-reliable VCTE), is to be considered as a false negative (worst scenario) in the six-cell matrix. Still in this example, the virtual stiffness value used would be 6.3kPa, which is the 25^{th} centile among the 347 evaluable VCTE-stiffness measures in men.

All statistical analyses were performed using R, version 4.0.3 or NCSS 2022.

### Example 3. Results

### Characteristics of patients

Between February 2018 and December 2022, 434 outpatients with suspected NAFLD were referred by diabetologists to hepatologists for further work-up (**Figure 1**). After exclusion of 32 patients without biopsy analyzed, the remaining 402 participants was the intention-to-diagnose subset, and the standard subset (n=347) including only participants with all nine NITs evaluable, 55 being excluded.

Characteristics of included patients were not significantly different compared to the excluded at the exception of less patients with BMI lower than 25 kg/m² and higher glucose medians. Median age was 59 years, 40% female, 80% European, 33% had BMI of 35 kg/m² or higher, 44% consumed tobacco and 67% had arterial hypertension. At inclusion, most patients were treated with several medications: oral antidiabetic (97%), cardiovascular (73%) and lipid lowering (65%) drugs.

As expected by the low limits of inclusion criteria, the median AST and ALT levels were moderately elevated 35 IU/L and 59 IU/L respectively. Median of carbohydrate-deficient-transferrin was not elevated in line with the exclusion of patients with an elevated alcohol consumption. The median HbA1c was 7.4% (>6.5% the consensual upper limit), correlating with poorly controlled diabetes.²⁹

Advanced fibrosis (F3-F6) was present in 39% of patients including 9% of cirrhosis (stages F4-F6). Advanced NASH (grades N3-N4) was present in 32% and advanced steatosis (grades S3-S4) in 74% of patients.

### Optimization of Nash-Fibro Test- T2D

The final logistic regressions for fibrosis and the optimization of FibroTest-T2D combined for each sex adjusted on the age, the same four serum components (Alpha-2-macroglobulin, GGT, ApoA1, Haptoglobin) for the optimized FibroTest with different weights and without the need of bilirubin.

For the NashTest-T2D optimization and in comparison with the previous NashTest (described in WO2018050804), the separate logistic regressions by sex adjusted on age, permitted to reduce the number of serum components from 10 to five in female with still A2M, GGT, bilirubin and AST but different weights and with the addition of HbA1c, and the exclusion of apoA1, haptoglobin, ALT and TGL. In male, the number of serum components was reduced from 10 to four, with different weights and without the need to add HbA1c.

For the SteatoTest-T2D optimized in comparison with previous SteatoTest (described in WO2018050804), the separate logistic regressions by sex adjusted on age permitted to reduce the number of serum components from 10 to nine in female with still A2M, GGT, haptoglobin, bilirubin, ALT, TGL and cholesterol, all with different weights and with the addition of BMI and Hb1A1c, with the exclusion of apoA1, AST and glucose. In male, the number of serum components was reduced from 10 to six, A2M, GGT, bilirubin, ALT, TGL and cholesterol, all with different weights, with the exclusion of apoA1, haptoglobin, AST and glucose. with still with different weights, and without the need of BMI and HbA1c.

### Performances of optimized NashFibroTest-T2D vs. previous NashFibroTest described in WO2018050804 or the Fibrotest^{®} described in WO0216949

The comparison between performances is detailed in Table 1.

For the diagnosis of advanced fibrosis stages F3-F6, the AUROC was improved by 0.066 (.028 - .104; P=.001), mostly due to higher sensitivity.

For the diagnosis of advanced Nash grades N3-N4, the AUROC of the optimized NashTest-T2D was improved by 0.054 (.015 - .093; P=.007) vs. previous NashTest, mostly due to higher specificity.

For the diagnosis of advanced steatosis grades S3-S4, the AUROC of the optimized SteatoTest-T2D was improved by .138 (.074 - .202) P=.0001) vs. previous SteatoTest, mostly due to higher specificity.

The formulas developed to detect NASH within the NashFibroTest-T2D (NashTest-T2D) are:
- 11.5677 + 0.0215*Age (years) + 4.4354*Log(AST, IU/L) + 4.3850*Log(1 + A2m, g/L) + 0.2583*(Hb1Ac, %) - 0.7781*Log(Bili, µmol/L), when the subject is a female and
- 11.6213 + 0.0024* Age (years) + 3.0409* Log(AST, IU/L) + 1.4619*Log(GGT, IU/L) + 2.1631*Log(1 + A2m, g/L) + 0.6593*Log(Bili, µmol/L) + 0.2872*(Hb1Ac, %) when the subject is a male.

The results are then normalized between 0 and 1. Table 1 shows a better AUROC, with increased Specificity and Positive predictive value when the cutoff (predetermined value) is set at 0.5.

**Table 1. Diagnostic performances of optimized NashFibroTest-T2D vs original NashFibroTest (described in WO2018050804)**

| | **NashFibroTest-T2D** Mean (95% CI) | **NashFibroTest** Mean (95% CI) | **Paired difference between AUROCs** (95%CI) and p-value significance |
|---|---|---|---|
| **FibroTest for F3-F6** | Cutoff =.5 Prevalence F3-F6 = .39 | | **.066 (.028 - .104)** |
| | | | **P=.001** |
| **AUROC n=402** | **.774 (.725-.816)** | **.709 (.654.756)** | |
| Sensitivity | .699 (.620-.769) | .468 (.388-.549) | |
| Negative predictive value | .786 (.726-.839) | .700 (.643-.754) | |
| Specificity | .703 (.642-.760) | .789(.732-.838) | |
| Positive predictive value | .599 (.524-.671) | .584 (.492-.671) | |
| **NashTest for N3-N4** | Cutoff=.5 Prevalence N3-N4= .31 | | **.054 (.015 - .093)** |
| | | | **P=.007** |
| **AUROC n=390** | **.771 (.717-.816)** | **.717 (.659-.767)** | |
| Sensitivity | .669 (.578-.752) | .975 (.929-.995) | |
| Negative predictive value | .828 (.773-.874) | .947 (.854-0.989 | |
| Specificity | .714 (.656-.767) | .201 (.155-.254) | |
| Positive predictive value | .513 (.432-.593) | .354 (.303-.408) | |
| **SteatoTest for S3-S4** | cutoff=.5 | | **.138 (.074 - .202) P=.0001** |
| **AUROC n=400** | **.753 (.692-.804)** | **.615 (.569-.693)** | |
| Sensitivity | .679 (.623-.732) | .909 (.870-.939) | |
| Negative predictive value | .421 (.344-.500) | .341(.201-.506) | |
| Specificity | .663 (.564-.753) | .135 (.076-.216) | |
| Positive predictive value | .852 (.800-.894) | .749 (.701-.793) | |

### Performance of NITs according to intention-to-diagnose versus standard analysis

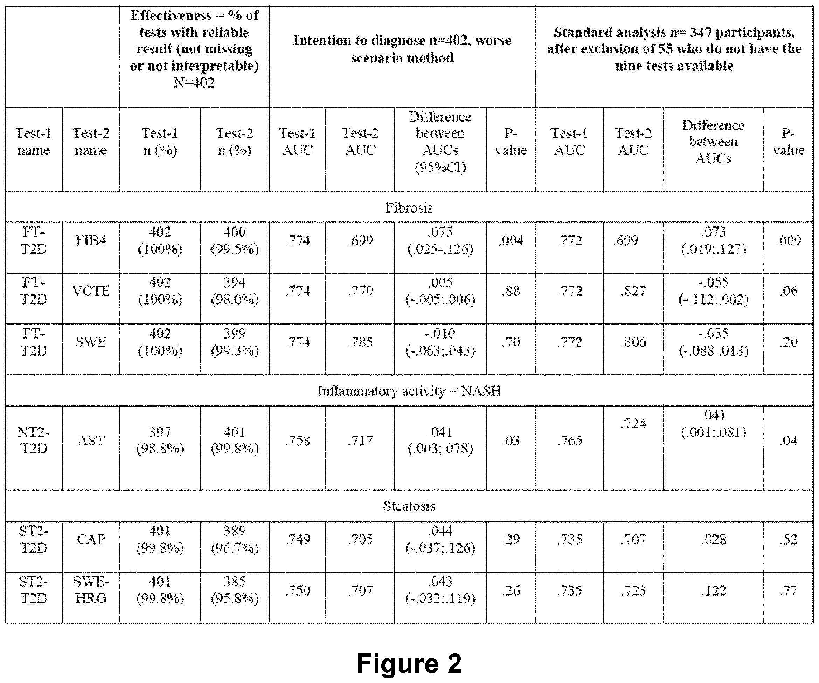

For advanced fibrosis stages >=F3 the binary AUROC of VCTE was reduced from .827 (95%CI .778- .866) in standard analysis to .770 (95%CI .719-.813;SE=.024;SD=.155) in intention-to-diagnose, a 6.9% (57/827) significant difference (P= 0.01) (Figure 2).

Comparison with the Fibrotic NASH" [FINI Index] also showed a better AUROC for significant fibrosis and advanced activity.

### Example 4. Discussion

Non-alcoholic fatty liver disease (NAFLD), is the most common chronic liver disease, associated with hepatic mortality and morbidity, particularly in patients with type-2 diabetes (T2D).

Among the three main liver features of NAFLD, only advanced fibrosis stages are independently associated with mortality and recognized as the main endpoint for identifying the patients at risk of significant morbidity.

The grade of non-alcoholic steatohepatitis (NASH) is associated with a more rapid progression of fibrosis but is not independently associated with mortality after adjustment on fibrosis stage.

Steatosis is a sensitive and the earlier feature of NAFLD, but in T2D its prevalence is reduced when fibrosis stages increased.

NAFLD is underdiagnosed in real-world clinical settings despite the numerous NITs available. Studies indicate a high demand for diagnosis and monitoring tests in NAFLD. Differences in use of different diagnostic tests were also seen depending on country, with higher rates of liver biopsy, Nash-FibroTest, NAFLD fibrosis score, NAFLD activity score, enhanced liver fibrosis score and less frequent use of FibroScan and some laboratory tests (e.g. GGT, lipid profile) reported in the US compared with European countries.

Guidelines recommend screening for NAFLD in patients with T2D due to an estimated prevalence of around 38% for at least bridging fibrosis (stage F3 using NASH Clinical Research Network [CRN] or Steatosis-Activity-Fibrosis [SAF] scoring systems) in comparison with 22% in NAFLD without T2D.

However, there is still a controversy between the diagnostic performances of NITs in patients with T2D in comparison with more consensual performances in other chronic liver diseases such as viral hepatitis and alcoholic liver disease. Several methodological limitations already identified at least since 1982, but rarely applied which might explain most of these differences.

The first limitation is the small number of direct head-to-head comparisons between NITs. Also, these indirect comparisons assumed that the gold-standard is binary. But fibrosis stages, steatosis and NASH grades are offering several stages/grades. Indirect comparisons should be adjusted on the liver lesions' prevalence in the context of use and on the spectrum of stages or grades (spectrum effect).

The use of Obuchowski measure is recommended as well as adjusted-AUROC (area under the receiver operating curve) according to the prevalences of stages. Among 64 studies of fibrosis biomarkers in NAFLD, only four (6.4%) used Obuchowski measure. When compared using Obuchowski measures, the performances of NITs were not different in patients with or without T2D. Misleading conclusions are also observed in overviews of NITs performances when AUROCs are indirectly compared. For instance, a recent study in T2D only (n=220) found an AUROC of 0.720 for a blood test for advanced fibrosis stages F3F4, and concluded that this NIT "underperformed in T2D" (Bril et al, J Investig Med. 2019;67:303-311).

Indeed, a simple adjustment according to the spectrum of fibrosis of this population increased the AUROC by 7.5% to 0.774 (Poynard et al, Eur J Gastroenterol Hepatol. 20:998-1007).

The second limitation is the small number of studies analyzing the NITs' performance with the recommended intention-to-diagnose methodology. Among 84 studies of elastography and magnetic resonance imaging in patients with NAFLD only three (3.6%) used intention-to-diagnose and therefore the clinical utility could not be fully assessed.

The third limitation is the absence of prospective validation of pre-defined cut-offs. It is difficult to know how clinicians could apply the tests, as current society guidelines gave no specific cut-off recommendations.

Due to these unmet needs, we aim to compare head-to-head the diagnostic performance of nine widespread NITs, five blood NITs and four imaging NITs, prospectively, in outpatients with T2D using intention-to-diagnose analysis and for each of the three main histological features, with predetermined cut-offs, for the diagnosis of advanced fibrosis, NASH and steatosis.

The most recent reviews of NITs in T2D concluded unanimously that there is an unmet need to perform specific prospective studies in outpatients with T2D, using head-to-head comparison, to compare the numerous blood and imaging tests already available in subjects at risk of NAFLD.

As already underlined these overviews, the results confirm the importance for accurate comparisons between tests assessing the severity of liver injury in patients with NAFLD, to take into account three frequent limitations.

Firstly more prospective head-to-head comparisons studies must be done in outpatients with T2D. Secondly studies should be analyzed in intention-to-diagnose, and thirdly studies should reported accuracy against pre-specified cut-offs.

Here, we observed that using head-to-head comparisons several conclusions on biomarkers were not the same when these limitations were taken into account. For advanced fibrosis >= Stage F3 the binary AUROC of VCTE was significantly overestimated in standard analysis reduced from .83 (95%CI .78-.87) to .77 (95%CI .72-.81; P= 0.01) in intention-to-diagnose.

Interestingly the largest individual patient data meta-analysis in NAFLD (33% T2D, 30% >=F3 fibrosis stage) (Poynard et al, PLoS One. 2016;11:e0163276), found similar AUROCs using standard analysis, 0.85 (95%CI 0.84-0.86) for VCTE (n=5,489) and 0.75 (95%CI 0.71-0.75) for FIB4 (n=5,393), and in the subset of patients with head-to-head comparisons (n=3,248), the AUROCs were 0.86 (0.85-0.88) for VCTE and 0.73 (0.71-0.75) for FIB4. According to the 12% of non-reliable stiffness, the performance of VCTE in intention-to-diagnose the AUROC should be lower.

Thus, the results reported here showed that designing NITs tests specific for T2D patients provides as good results as other types of non-invasive tests, but that the combination of the various tests described herein provide a complete toolbox for the physician for conducting a full and complete diagnosis of a T2D patient with easily available and generally studied markers.

**Table 2. Optimized FibroTest, NashTest and SteatoTest, for patients with T2D. Logistic regression and comparison of AUROCs by gender.**

| | **Optimized NashFibroTest forT2D** | | **Regular NashFibroTest** | |
|---|---|---|---|---|
| | **Female n=159** | **Male n=243** | **Female n=159** | **Male n=243** |
| **Fibrosis** | | | | |
| Test name | FibroTest-T2D | FibroTest-T2D | FibroTest | FibroTest |
| Components | n=5 | n=5 | n=6 | n=6 |
| Age | yes | yes | yes | yes |
| A2M | yes | yes | yes | yes |
| GGT | yes | yes | yes | yes |
| ApoA1 | yes | yes | yes | yes |
| Haptoglobin | yes | yes | yes | yes |
| Bilirubin | no | no | yes | yes |

| **Nash** | | | | |
|---|---|---|---|---|
| Test name | NashTest-T2D | NashTest-T2D | NashTest | NashTest |
| Components | n=5 | n=6 | n=11 | n=11 |
| Age | yes | yes | yes | yes |
| A2M | yes | yes | yes | yes |
| GGT | no | yes | yes | yes |
| ApoA1 | no | no | yes | yes |
| Haptoglobin | no | no | yes | yes |
| Bilirubin | yes | yes | yes | yes |
| ALT | no | no | yes | yes |
| AST | yes | yes | yes | yes |
| TGL | no | no | yes | yes |
| HbA1c | yes | yes | no | no |

| **Steatosis** | | | | |
|---|---|---|---|---|
| Components | n=9 | n=6 | n=11 | n=11 |
| Age | yes | yes | yes | yes |
| A2M | no | no | yes | yes |
| GGT | no | yes | yes | yes |
| ApoA1 | yes | yes | yes | yes |
| Haptoglobin | yes | no | yes | yes |
| Bilirubin | yes | yes | yes | yes |
| ALT | yes | yes | yes | yes |
| AST | no | no | yes | yes |
| TGL | yes | yes | yes | yes |
| BMI | yes | no | no | no |
| HbA1c | yes | no | no | no |
| Glucose | no | no | yes | yes |
| Cholesterol | yes | yes | yes | yes |

## Claims

1. An *in vitro* method for diagnosing the presence of Non-Alcoholic Steatotic Hepatitis (NASH) in a subject, wherein the subject has Type-2 Diabetes (T2D) comprising:
(a) Determining the genetic sex of the subject
(b) Obtaining the age in years of the subject
(c) Obtaining the values of biomarkers measured in the blood, plasma or serum of the subject, wherein the biomarkers are aspartate transaminases (AST), bilirubin (BILI), alpha2-macroglobulin (A2M), Glycated hemoglobin (HbAc1),
(d) Additionally obtaining the values
∘ of biomarker gammaglutamyl transpeptidase (GGT) when the subject is a male
(e) optionally obtaining values of other circulating biomarkers
(f) Combining the values obtained in (b), (c) (d) and (e) in a function,
(g) obtaining an output from (f)
(h) optionally comparing the output to predetermined values, and determining presence of NASH based on the end value calculated in (g) and the comparison to the predetermined values

2. The method of claim 1, wherein , wherein the function the values that are combined in the function of (f) are values for aspartate transaminases (AST), bilirubin (BILI), alpha2-macroglobulin (A2M), Glycated hemoglobin (HbAc1) and age when the subject is a female and aspartate transaminases (AST), bilirubin (BILI), alpha2-macroglobulin (A2M), Glycated hemoglobin (HbAc1), gammaglutamyl transpeptidase (GGT) and age when the subject is a male.

3. The method of claim 1 or 2, wherein the function is
∘ a1 + a2*Age (years) + a3*Log(AST, IU/L) + a4*Log(1 + A2m, g/L) + a5*(Hb1Ac, %) + a6*Log(Bili, µmol/L) if the subject is a female, or
∘ b1 + b2* Age (years) + b3* Log(AST, IU/L) + b4*Log(GGT, IU/L) + b5*Log(1 + A2m, g/L) + b6*Log(Bili, µmol/L) + b7*(Hb1Ac, %) if the subject is a male
wherein
▪ -12.0≤ a1 ≤-11.0, preferably -11.7≤ a1 ≤-11.3
▪ 0.015≤ a2 ≤0.025, preferably 0.018≤ a2 ≤0.024
▪ 4.30≤ a3 ≤4.60, preferably 4.40≤ a3 ≤4.50
▪ 4.30≤ a4 ≤4.50, preferably 4.30≤ a4 ≤4.45
▪ 0.17≤ a5 ≤0.32, preferably 0.20≤ a5 ≤0.30
▪ -0.90≤ a6 ≤-0.70, preferably -0.82≤ a6 ≤-0.72
▪ -12.0≤ b1 ≤-11.0, preferably -11.8≤ b1 ≤-11.4
▪ 0.0015≤ b2 ≤0.0035, preferably 0.002≤ b2 ≤0.003
▪ 2.90≤ b3 ≤3.20, preferably 3.0≤ b3 ≤3.1
▪ 1.35≤ b4 ≤1.55, preferably 1.4≤ b4 ≤1.5
▪ 2.0≤ b5 ≤2.3, preferably 2.1≤ b5 ≤2.2
▪ 0.60≤ b6 ≤0.75, preferably 0.62≤ b6 ≤0.70
▪ 0.22≤ b7 ≤0.34, preferably 0.25≤ b7 ≤0.30

4. The method of any one of claims 1 to 3, wherein the function is -11.5677 + 0.0215*Age (years) + 4.4354*Log(AST, IU/L) + 4.3850*Log(1 + A2m, g/L) + 0.2583*(Hb1Ac, %) - 0.7781*Log(Bili, µmol/L), when the patient is a female and -11.6213 + 0.0024* Age (years) + 3.0409* Log(AST, IU/L) + 1.4619*Log(GGT, IU/L) + 2.1631*Log(1 + A2m, g/L) + 0.6593*Log(Bili, µmol/L) + 0.2872*(Hb1Ac, %) when the patient is a male.

5. The method of any one claims 1 to 4, wherein the functions have been obtained by:
a) evaluating the presence of NASH in a cohort of patients with type-2 diabetes, wherein the values of circulating biochemical markers are known for the patients, and wherein the age and sex are also known
b) identifying by unidimensional analysis, the circulating biochemical markers for which the values differ significantly
i. between the groups of male patients with NASH and male patients without NASH and/or
ii. between the groups of female patients with NASH and female patients without NASH
c) performing a regression analysis (generally a logistic regression) for each group (male and female patients) to assess and ponder the independent discriminative value of the markers identified in step b) for the occurrence of steatosis in each (male or female) studied group
d) thereby obtaining a function for male patients and/or a function for female patients, by combination of these identified independent factors.

6. The *in vitro* method of any one of claims 1 to 6, further comprising normalizing the output to obtain a final value comprised between 0 and 1.

7. The method of claim 7, wherein the subject does not have NASH if the final value is below 0.25, and wherein the subject has NASH if the final value is higher or equal to 0.5.

8. A method for obtaining a function for identifying the presence of NASH in a subject with type 2 diabetes (T2D), wherein said function combines the values of the concentration of biochemical markers in the blood / serum or plasma of said patient and of the age of the subject, wherein the function is specific for subjects of a given biological gender, comprising the steps of:
a) evaluating the presence of NASH in a cohort of patients with type-2 diabetes, wherein the values of circulating biochemical markers are known for the patients, wherein the cohort of patients contains only male patients or only female patients
b) identifying by unidimensional analysis, the circulating biochemical markers for which the values differ significantly between the groups of
i. patients with NASH and
ii. patients without NASH
c) performing a logistic regression analysis to assess and ponder the independent discriminative value of the markers identified in step b) and of the age of the patients for the occurrence of steatosis,
d) thereby obtaining the function, by combination of these identified independent factors, wherein the function allows for diagnosing the presence of NASH in a patient of the same sex than the one of the cohort.

9. An *ex vivo* method for diagnosis a liver disease in a T2D patient, comprising
- Performing the method of any one of claims 1 to 7 to determine presence of NASH in a patient, and
- Determining the presence of liver fibrosis in the patient, and/or
- Further determining the presence of liver inflammation or liver activity in the patient.

10. The method of claim 9, wherein liver fibrosis is determined by the Fibrotest^{®}, or by *ex vivo* measurement of liver stiffness by Vibration Controlled Transient Elastography.

11. The method of claim 9 or 10, wherein presence of liver inflammation or liver activity in the patient is determined by a non-invasive test.

12. The method of claim 9, wherein presence of liver fibrosis and/or of liver inflammation liver activity is determined by *ex vivo* analysis of samples from a biopsy of the patient's liver.

13. A computer-implemented method for diagnosing the presence of NASH in a subject, wherein the subject has Type-2 Diabetes (T2D) comprising
a) Receiving inputs from a sender, wherein the inputs are the values of biomarkers measured in the blood, serum or plasma of the subject, as well as the information pertaining to the age, height, weight and sex of the patient
b) Performing a method of any one of claims 1 to 7, to obtain an output for the combination of the values received in (a)
c) optionally comparing the output to a reference value and determining the presence of the NASH
d) Sending the output to the sender and/or the presence of NASH, if determined
e) Optionally storing the inputs and the output in a database.

14. A computer implemented method for obtaining information as to the presence of NASH in a subject, wherein the subject has Type-2 Diabetes (T2D) comprising
a) Sending inputs to a server, wherein the inputs are the values of biomarkers measured in the blood, serum or plasma of the patient, as well as the information pertaining to the age, sex, height and weight of the subject
b) Having the remote server combine the values received in (a) in a function, described in any one of claims 1 to 9, and obtain an output, optionally normalized
c) optionally having the remote server compare the output to a reference value and determining the presence or absence of NASH in the subject
d) Optionally having the remote server store the inputs and the output in a database
e) receiving the output from the server and/or the information pertaining to the presence or absence of NASH (when determined).

15. A computer-implemented method for determining whether a patient has NASH
a) requiring a sender to identify himself to a server within a public or private network,
b) requiring the sender to fill in information related to the patient, and assigning a specific identifier to the patient in a database, (this last step may be omitted if the patient has already been recorded in the database, as can be determined, using the patient information);
c) receiving values of the concentration of measured biological markers, in particular (AST, A2M, Hb1Ac, BILI, GGT, age (it may be calculated from the birth date), wherein the values are received in a secure manner, and storing the values in the database, associated with the specific identifier of the patient
d) combining the values received or calculated in c) by use of a function as disclosed in any one of claims 1 to 8, obtaining a result, and optionally normalizing the result
e) storing the result optionally normalized of d) in a database associated with the specific identifier and preferably the date and time of identification of the sender, thereby obtaining a database where the information related to the patient is present under the specific identifier,
f) sending the nature of the presence of NASH to the sender, wherein the patient has NASH if the result is above a predetermined value and the patient has not NASH if the result is below the or another predetermined value.
